# EUROPEAN PATENT SPECIFICATION

(11) **EP 1 818 057 B1**
(45) Date of publication and mention of the grant of the patent: **21.04.2010**
(21) Application number: 06252449.1
(22) Date of filing: 09.05.2006
(51) Int. Cl.: A61K 31/47, A61K 9/20, A61K 9/28, A61P 11/06

(54) **Stable pharmaceutical formulations of montelukast sodium**
Stabile pharmazeutische Zubereitungen von Montelukast-Natrium
Composition pharmaceutique stable contenant de montelukast de sodium

(30) Priority: 09.02.2006 US 772258 P
(43) Date of publication of application: 15.08.2007
(60) Divisional application: 09177374.7
(73) Proprietor: TEVA PHARMACEUTICAL INDUSTRIES LTD., 49131 Petah Tiqva (IL)
(72) Inventor: Hrakovsky, Julia, Rosh Ha-Ayin 48057 (IL); Tenengauzer, Ruth, Hod Hasharon 45322 (IL); Bogomolny, Grigory, Kefar-Sava 44391 (IL); Dolitsky, Yehudit, Petah Tiqva 49651 (IL)
(74) Representative: Russell, Tim

(56) References cited:
- WO-A-2005/074893
- CN-A- 1 628 666
- US-A- 5 565 473
- US-A1- 2005 107 426

## Description

### FIELD OF THE INVENTION

The invention encompasses stable pharmaceutical compositions in film coated tablet form comprising montelukast or salts thereof and methods of preparing the same. Preferably, the salt is the sodium salt.

### BACKGROUND OF THE INVENTION

Montelukast is apparently a selective, orally active leukotriene receptor antagonist that inhibits the cysteinyl leukotriene CysLT₁ receptor.

The chemical name for montelukast sodium is [R-(*E*)]-1-[[[1-[3-[2-(7-chloro-2-quinolinyl)ethenyl]phenyl]-3-[2-(1-hydroxy-1-methylethyl)phenyl]propyl]thio]methyl] cyclopropaneacetic acid, monosodium salt. Montelukast sodium salt is understood to be represented by the following structural formula:

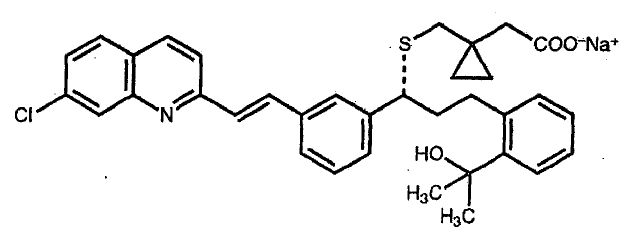

U.S. patent No. 5,565,473 ("'473 patent") is listed in the FDA's Orange Book for montelukast sodium. The '473 patent recites a broad class of leukotriene antagonists as "anti-asthmatic, anti-allergic, anti-inflammatory, and cycloprotective agents" represented by a generic chemical formula. '473 patent, col. 2,1. 3 to col. 4, 1. 4. Montelukast is among the many compounds represented by that formula. The '473 patent also refers to pharmaceutical compositions of the class of leukotriene antagonists of that formula with pharmaceutically acceptable carriers. *Id*. at col. 10, ll. 42-46.

WO2003/074893 describes amorphous montelukast sodium and co-precipitates containing the same. The document also describes pharmaceutical compositions comprising the same.

Montelukast sodium is currently marketed by Merck in the form of film coated tablets and chewable tablets under the trade name Singular^{®}. The film coated tablets reportedly contain montelukast sodium and the following inactive ingredients: microcrystalline cellulose, lactose monohydrate, croscarmellose sodium, hydroxypropylcellulose, magnesium stearate, titanium dioxide, red ferric oxide, yellow ferric oxide, and carnauba wax. The chewable tablets reportedly contain montelukast sodium and the following inactive ingredients: mannitol, microcrystalline cellulose, hydroxypropylcellulose, red ferric oxide, croscarmellose sodium, cherry flavor, aspartame, and magnesium stearate. Physicians' Desk Reference, 59th ed. (2005), p. 2141.

However, there is a need in the art to improve the stability of compositions of montelukast and particularly those of the sodium salt.

### SUMMARY OF THE INVENTION

One embodiment of the invention encompasses a stable pharmaceutical composition in film coated tablet form comprising montelukast or a salt thereof and a pharmaceutically acceptable excipient selected from at least one of diluent, binder, disintegrant, wetting agent, lubricant, and glidant, provided that the pharmaceutically acceptable excipient is not microcrystalline cellulose wherein the pharmaceutical composition is prepared by direct compression from a dry mix.

Preferably, the salt is montelukast sodium.

In an embodiment of the invention the pharmaceutical composition contains montelukast sodium and also contains the corresponding montelukast sulfoxide of formula (I): 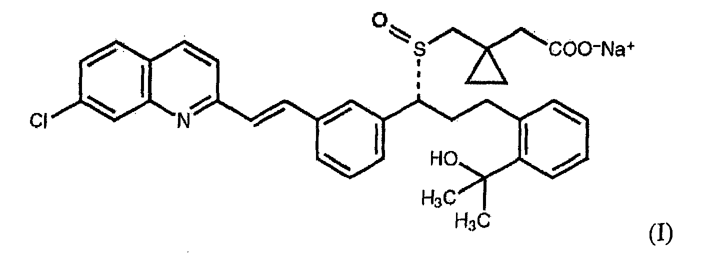 wherein the corresponding montelukast sulfoxide of formula (I) in the composition does not increase by more than 1% by weight from the initial amount of montelukast after storage at about 40°C and about 75% relative humidity for 3 months.

Preferably, the sulfoxide content has not increased by more than 0.5% by weight of the initial amount of montelukast after storage at about 40°C at about 75% relative humidity for 3 months. More preferably, the sulfoxide content has not increased by more than 0.3% by weight of the initial amount of montelukast after storage at about 40°C at about 75% relative humidity for 3 months. Most preferably, the sulfoxide content has not increased by more than 0.1 % by weight of the initial amount of montelukast after storage at about 40°C at about 75% relative humidity for 3 months.

In another embodiment of the invention, the pharmaceutical composition comprises montelukast sodium.

In another embodiment of the invention, immediately after preparation of the pharmaceutical composition, the corresponding sulfoxide is present in an amount of not more than 0.2% by weight of montelukast in the pharmaceutical composition.

Film coated tablets may comprise the pharmaceutical composition and a coating agent, provided that the coating agent is not microcrystalline cellulose. Preferably, the film coated tablets comprise montelukast sodium, lactose monohydrate, hydroxypropylcellulose, starch, sodium starch glycolate, magnesium stearate, and a coating. The coating may be made from a commercially available powder mix for preparing coating suspensions such as Opadry^{®}. Opadry®, available from Colorcon, has hydroxypropyl cellulose, hypromellose, titanium dioxide, and iron oxide. More preferably, the film coated tablet comprises about 5% by weight montelukast sodium, about 62% by weight lactose, about 2% by weight hydroxypropylcellulose, about 18% by weight starch, about 9% by weight sodium starch glycolate, about 1% by weight magnesium stearate, and about 3% by weight Opadry^{®}. The ordinarily skilled artisan will recognize that the coating can be prepared from the constituent elements rather than the commercially available premixed preparation.

### DETAILED DESCRIPTION

Montelukast compositions are subject to degradation during manufacture and storage. It is believed that the montelukast degrades into its corresponding sulfoxide. The sulfoxide is an inactive impurity, which reduces the effective dosage of montelukast when it is administered to a patient. The present invention overcomes this problem by providing compositions of montelukast that are stable to this degradation.

As used herein, unless otherwise defined, the term "corresponding sulfoxide" refers to montelukast or a salt thereof wherein the sulfide group in the β-position relative to the cyclopropane group has been oxidized to a sulfoxide group, e.g. the sulfoxide of formula (I) wherein the montelukast salt is montelukast sodium has the formula:

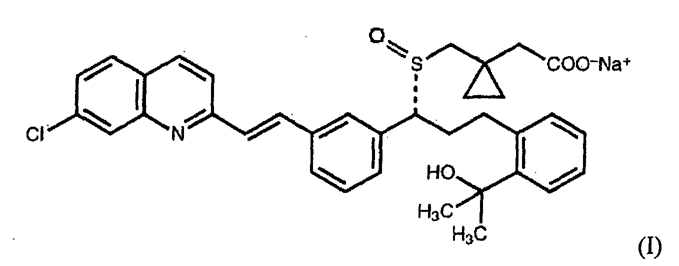

As used herein with respect to pharmaceutical compositions, unless otherwise defined, the term "stable" means that the amount of the corresponding sulfoxide within the montelukast in the packaged pharmaceutical composition has not increased by more than 1% by weight from the initial amount of montelukast after storage at about 40°C and about 75% relative humidity for 3 months. Preferably, the corresponding sulfoxide content has not increased by more than 0.5% by weight of the initial amount of montelukast after storage at about 40°C at about 75% relative humidity for 3 months. More preferably, the corresponding sulfoxide content has not increased by more than 0.3% by weight of the initial amount of montelukast after storage at about 40°C at about 75% relative humidity for 3 months. Most preferably, the corresponding sulfoxide content has not increased by more than 0.1 % by weight of the initial amount of montelukast after storage at about 40°C at about 75% relative humidity for 3 months.

As used herein, unless otherwise defined, the term "accelerated storage conditions" refers to storage of montelukast at about 40°C at about 75% relative humidity for 3 months.

As used herein unless otherwise defined, the term "immediately after preparation," as applied to formulations, means the time elapsed from the preparation of the formulation and not exceeding 48 hours.

Comparative testing with montelukast sodium was performed to determine the conditions which cause the degradation of montelukast sodium into the corresponding sulfoxide. Compositions of montelukast sodium and each of the excipients of the prior art tablets were prepared and subjected to stressed storage conditions. The amount of the corresponding sulfoxide present in each of the compositions was measured by high performance liquid chromatography ("HPLC") both before and after storage. It was found that the amount of the corresponding sulfoxide in the composition increased by over 250% in the presence of microcrystalline cellulose over the storage period.

Not to be limited by theory, it is believed that the poor stability of the prior art compositions of montelukast can be attributed to the presence of microcrystalline cellulose. Microcrystalline cellulose may contain peroxide, which can catalyze the conversion of montelukast to its corresponding sulfoxide. For example, montelukast sodium may degrade into the sulfoxide of formula (I).

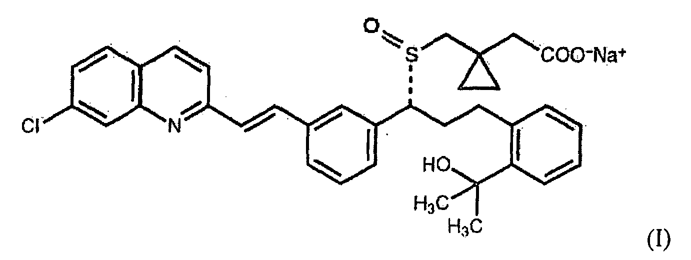

One embodiment of the invention encompasses stable pharmaceutical compositions in film coated tablet form comprising montelukast or a salt thereof and a pharmaceutically acceptable excipient selected from at least one of diluent, binder, disintegrant, wetting agent or lubricant, provided that the pharmaceutically acceptable excipient is not microcrystalline cellulose wherein the pharmaceutical composition is prepared by direct compression form a dry mix. Optionally, the pharmaceutical compositions of the invention further comprise at least one coating agent, sweetening agent, flavoring agent, coloring agent, or glidant.

Diluents increase the bulk of a solid pharmaceutical composition, and may make a pharmaceutical dosage form containing the composition easier for the patient and care giver to handle. Diluents used in the composition include diluents commonly used in solid pharmaceutical compositions. Diluents include, but are not limited to, calcium carbonate, calcium phosphate (dibasic or tribasic), calcium sulfate, dextrates, dextrin, dextrose excipient, fructose, kaolin, lactitol, anhydrous lactose, lactose monohydrate, maltose, mannitol, sorbitol, sucrose, starch, pregelatinized starch, or talc. Preferably, the diluent is at least one of lactose monohydrate, starch, or mannitol. Typically, the diluent is present in an amount of about 60% to about 95% by weight of the composition.

Binders help to bind the active ingredient and other excipients together. Binders used in the composition include binders commonly used in solid pharmaceutical compositions. Binders include, but are not limited to, acacia, alginic acid, carbomer, sodium carboxymethylcellulose, dextrin, ethylcellulose, gelatin, glucose, guar gum, hydroxypropylcellulose, maltose, methylcellulose, povidone, starch, methylcellulose, or polyethylene oxide. Preferably, the binder is hydroxypropylcellulose. Typically, the binder is present in an amount of about 1% to about 5% by weight of the composition.

Disintegrants increase the dissolution rate of a solid pharmaceutical composition in the patient's body. Disintegrants used in the composition include disintegrants commonly used in solid pharmaceutical compositions. Disintegrants include, but are not limited to, alginic acid, croscarmellose sodium, crospovidone, potassium polacrilin, sodium starch glycolate, and starch. Preferably, the disintegrant is at least one of sodium starch glycolate or starch. Typically, the disintegrant is present in an amount of about 5% to about 15% by weight of the composition.

Wetting agents are added to pharmaceutical compositions for facilitating processing. Wetting agents used in the composition include wetting agents commonly used in solid pharmaceutical compositions. Wetting agents used in the composition include, but are not limited to, sodium lauryl sulfate. Generally, suitable wetting agents can be selected by the method outlined in example 1 below, to ensure that they have no excessive adverse effect on the stability of the montelukast.

Lubricants are added to a pharmaceutical composition for ease in processing, to prevent adhesion to the equipment used during processing. Lubricants used in the composition include lubricants commonly used in solid pharmaceutical compositions. Lubricants used in the composition include, but are not limited to, calcium stearate, glyceryl behenate, magnesium stearate, mineral oil, polyethylene glycol, sodium stearyl fumarate, stearic acid, talc, vegetable oil, sodium lauryl sulfate, or zinc stearate. Preferably, the lubricant is magnesium stearate. Typically, the lubricant is present in an amount of about 0.5% to about 2% by weight of the composition.

Coating agents facilitate the administration of a solid pharmaceutical composition to a patient, by making it easier for a patient to swallow the composition. Coating agents used in the composition include coating agents commonly used in solid pharmaceutical compositions. Coating agents include, but are not limited to, sodium carboxymethylcellulose, cellulose acetate, cellulose acetate, phthalate, ethylcellulose, gelatin, pharmaceutical glaze, hydroxypropylcellulose, hydroxypropylmethylcellulose, hypromellose phthalate, methacrylic acid copolymer, methylcellulose, polyethylene glycol, polyvinyl acetate phthalate, shellac, sucrose, titanium dioxide, lactose, or carnauba wax. The coating may also contain a coloring agent. Preferably, the coating agent is Opadry^{®}, which is a commercially available coating material prepared by Colorcon and contains hydroxypropyl cellulose, hypromellose, titanium dioxide, and iron oxide. The ordinary practitioner will recognize that this coating agent may be prepared from these ingredients rather than the commercially available premixed Opadry^{®} preparation, without departing from the scope of the invention. Typically, the coating agent is present in an amount of about 1% to about 3% by weight of the composition.

Sweetening agents are used to sweeten pharmaceutical compositions. Sweetening agents used in the composition include sweetening agents commonly used in solid pharmaceutical compositions. Sweetening agents include, but are not limited to, aspartame, dextrates, dextrose, fructose, mannitol, saccharin, sorbitol, sucralose, sucrose, sugar, or syrup. Preferably, the sweetening agent is at least one of aspartame or mannitol. When aspartame is used, care must be taken to use a minimal amount so as not to effect an interaction with the active ingredient in the chewable tablets of the invention; hence the amount should be about 1 mg per tablet or less. Typically, the sweetening agent is present in an amount of about 0.5% to about 90% by weight of the composition.

Flavoring agents make a pharmaceutical composition more palatable to the patient. Flavoring agents used in the composition include flavoring agents commonly used in solid pharmaceutical compositions. Flavoring agents used in the composition include, but are not limited to, maltol, vanillin, ethyl vanillin, menthol, citric acid, fumaric acid, ethyl maltol, tartaric acid, peppermint, artificial or natural fruit flavors. Typically, the flavoring agent is present in an amount of about 1% to about 3% by weight of the composition.

Coloring agents improve the appearance of a pharmaceutical composition and/or facilitate patient identification of the composition. Coloring agents used in the composition include coloring agents commonly used in solid pharmaceutical compositions. Coloring agents used in the composition include, but are not limited to, caramel, ferric oxides (red, yellow, or black), or natural or synthetic organic colors and lakes. Preferably, the coloring agent is ferric oxide. Typically, the coloring agent is present in an amount of about 0.1 % to about 1% by weight of the composition.

Glidants improve the flowability of a non-compacted solid composition and improve the accuracy of dosing. Glidants used in the composition include glidants commonly used in solid pharmaceutical compositions. Glidants used in the composition include, but are not limited to, colloidal silicon dioxide, magnesium trisilicate, starch, talc, or tribasic calcium phosphate. Preferably, the glidant is colloidal silicon dioxide. Typically, the glidant is present in an amount of about 0.3% to about 1.5% by weight of the composition.

In one preferred embodiment of the invention, the pharmaceutical compositions comprise montelukast sodium and a pharmaceutically acceptable excipient selected from at least one of diluent, binder, disintegrant, or lubricant, provided that the pharmaceutically acceptable excipient is not microcrystalline cellulose. Optionally, the pharmaceutical compositions further comprise at least one coating agent, sweetening agent, flavoring agent, coloring agent, or glidant.

The pharmaceutical composition in tablet form is a film coated tablet. Preferably, the film coated tablet comprises montelukast sodium, diluent, binder, disintegrant, lubricant, and coating agent, with the proviso that the tablet does not comprise microcrystalline cellulose. More preferably, the film coated tablet comprises montelukast sodium, lactose monohydrate, hydroxypropylcellulose, starch, sodium starch glycolate, magnesium stearate, and Opadry^{®}. Most preferably, the film coated tablet comprises about 5% by weight montelukast sodium, about 62% by weight lactose monohydrate, about 2% by weight hydroxypropylcellulose, about 18% by weight starch, about 9% by weight sodium starch glycolate, about 1% by weight magnesium stearate, and about 3% by weight Opadry^{®}.

Although the use of the suggested excipients should result in stable compositions, there may be variability within the commercially available grades and types of excipients, and impurities that might be present in an excipient from a particular source. A test protocol similar to that described in Example 1 can determine if a particular excipient is a source for instability.

The solid pharmaceutical dosage forms of the invention is prepared by direct compression of the formulation into tablets.

Typically, the film coated tablets are prepared by dry blending. For example, the blended composition of the active ingredients and excipients may be compacted into a slug or a sheet and then comminuted into compacted granules. The compacted granules may subsequently be compressed into a tablet, typically with the addition of a lubricant.

Preferably, montelukast sodium is blended with diluents and binders to form a blend. Disintegrant is then added to the blend and blended. Lubricant is then added to the blend and blended. The blend is compressed into a tablets and coated with coating agent to form film coated tablets.

Having described the invention with reference to certain preferred embodiments, other embodiments will become apparent to one skilled in the art from consideration of the specification. The invention is further defined by reference to the following examples describing in detail methods for the preparation and testing of the montelukast pharmaceutical compositions. It will be apparent to those skilled in the art that many modifications, both to materials and methods, may be practiced without departing from the scope of the invention.

### EXAMPLES

### Example 1:

Sample mixtures of montelukast sodium and each of the individual excipients were prepared. The composition of each of the samples is listed in Tables 1a and 1b. Each sample was stored at 55°C for 48 hours. The percentage by weight of sulfoxide of formula (I) relative to montelukast sodium in each sample was measured at 0 hours and at 48 hours by HPLC. HPLC was performed on a Beta-Basic C18 analytical column (150x4.6 mm I.D.), packed with 5µm diameter particles (Thermo Election Corporation). The mobile phase was a mixture of acetonitrile and 20 mM KH₂PO₄ (60:40) and its flow-rate was 1.5 mL/min. The UV detector was set at 225 nm or 281 nm and the column temperature was 40°C. The results are shown in Table 1.

**Table 1: Analysis of Montelukast Sodium Compositions with Various Excipients**

| Sample | Composition | % Sulfoxide of Formula (I) | |
|---|---|---|---|
| | | 0 hours | 48 hours |
| 1 | Montelukast sodium (a) | 0.63 | - |
| 2 | Montelukast sodium (1 g) / microcrystalline cellulose (10.1 g) | 0.59 | 1.52 |
| 3 | Montelukast sodium (1.5 g) / lactose (9.5 g) | 0.60 | 0.63 |
| 4 | Montelukast sodium (3.5 g) / hydroxypropylcellulose (7.4 g) | 0.58 | 0.62 |
| 5 | Montelukast sodium (7.5 g) / crospovidone (3.6 g) | 0.69 | 0.68 |
| 6 | Montelukast sodium (9.4 g) / magnesium stearate (1.8 g) | 0.56 | 0.57 |
| 7 | Montelukast sodium (b) | 0.26 | - |
| 8 | Montelukast sodium (1 g) / mannitol (36 g) | 0.25 | 0.28 |
| 9 | Montelukast sodium (2 g) / aspartame (2 g) | 0.24 | 0.73 |
| 10 | Montelukast sodium (2 g) / aerosol (2 g) | 0.25 | 0.25 |
| 11 | Montelukast sodium (2 g) / microcrystalline cellulose (20 g) / crospovidone (8 g) | 0.74 | 1.2 |

As illustrated by Table 1, the presence of microcrystalline cellulose in the composition caused a substantial increase in the amount of the sulfoxide of formula (I) upon storage. The amount of sulfoxide of formula (I) also increased in the presence of aspartame, although from a taste perspective, it may have to be included in the chewable tablets at a low level. Typically, aspartame should be present in an amount of not more than about 1 mg per tablet in order to achieve the desired stability. One may also be able to substitute mannitol for aspartame to improve stability, while preserving taste. There was no substantial change in the amount of the sulfoxide of formula (I) upon storage in the presence of the other excipients.

### Example 2 (Reference)

A pharmaceutical composition of montelukast sodium 10 mg tablets was prepared by a wet granulation method. Montelukast sodium (39.52 g), hydroxypropyl cellulose (15.2 g), crospovidone (76.0 g) and lactose (659.68 g) was mixed. The mixture was then granulated using purified water as a granulating liquid to form a granulate. The granulate was dried, milled and blended with magnesium stearate (7.6 g) to form a final blend. The final blend was compressed into the 10 mg tablets.

### Example 3:

A pharmaceutical composition of montelukast sodium was prepared by a dry mix method. A mixture was made of montelukast sodium (1352 g), lactose monohydrate (17303 g), and hydroxypropylcellulose (520 g). The mixture was blended for 20 minutes to form a blend. Starch (5200 g) and sodium starch glycolate (2600 g) were then added to the blend and blended for 10 minutes. Subsequently, magnesium stearate (325 g) was added to the blend and blended for an additional 5 minutes. The blend was compressed into tablets. The tablets were film coated using Opadry^{®} (780 g).

### Example 4:

A pharmaceutical composition of montelukast sodium was prepared by a dry mix method. A mixture was made of montelukast sodium (52 g), lactose monohydrate (634 g), and hydroxypropylcellulose (20 g). The mixture was blended for 20 minutes to form a blend. Starch (200 g), sodium lauryl sulfate (31.5g) and sodium starch glycolate (100 g) were then added to the blend and blended for 10 minutes. Subsequently, magnesium stearate (12.5 g) was added to the blend and blended for an additional 5 minutes. The blend was compressed into tablets.

| Composition | % Sulfoxide of Formula (I) | |
|---|---|---|
| | 0 hours | 72 hours at 55 degrees |
| Example 4 | 0.1 | 0.1 |

### Example 5: (Reference)

A pharmaceutical composition of montelukast sodium chewable tablets was prepared by wet granulation. A mixture was made of montelukast sodium (41.6 g), hydroxypropyl cellulose (40 g), sodium starch glycolate (80 g), mannitol (1490.4 g), color iron oxide (4 g), aspartame (8 g), and flavor (32 g). The mixture was then granulated using purified water as a granulating liquid to form a granulate. The granulate was dried, milled and blended with magnesium stearate (24 g) to form a blend. The blend was compressed into chewable tablets.

### Example 6: (Reference)

A pharmaceutical composition of montelukast sodium chewable tablets was prepared by a dry mix direct compression method. A mixture was made of montelukast sodium (23.4 g), hydroxypropylcellulose (22.5 g), sodium starch glycolate (45 g), aspartame (4.5 g), color (4.5 g), mannitol (858.6 g) and cherry flavor (18 g) and the mixture was blended for 15 minutes. Subsequently, magnesium stearate (13.5 g) was added to the blend and blended for an additional 5 minutes. The blend was compressed into chewable tablets.

### Example 7:

The pharmaceutical compositions prepared in Examples 3 and 5 were exposed to accelerated storage conditions, i.e., storage at about 40°C and about 75% relative humidity. The percentage by weight of sulfoxide of formula (I) relative to montelukast sodium in each sample was measured by HPLC immediately after the compositions were prepared and after 1, 2 and 3 months under accelerated storage conditions. HPLC was performed on a YMC-Pack ODS-AQ analytical column (100x4.6 mm I.D.), packed with 3µm diameter particles (YMC SEPARATION TECHNOLOGY). The mobile phase was a mixture of acetonitrile and 20 mM KH₂PO₄ at pH 2.0 (60:40) and its flow-rate was 1.0 mL/min. The UV detector was set at 285 nm and the column temperature was 30°C. Samples of Singular^{®} were also analyzed under the same conditions. The results are shown in Table 2.

**Table 2: Stability of Montelukast Sodium Compositions Under Accelerated Storage Conditions**

| Sample | % Sulfoxide of Formula (I) | | | |
|---|---|---|---|---|
| | Initial | 1 month | 2 months | 3 months |
| Composition of Example 3 | <0.1% | 0.1% | <0.1% | <0.1% |
| Composition of Example 5 | 0.1 % | 0.2% | 0.2% | - |
| Singular^{®}, 10 mg tablet | 0.2% | - | - | 0.3% |
| Singular^{®}, 5 mg tablet | 0.49% | - | - | - |

The percentage by weight of sulfoxide of formula (I) relative to montelukast sodium in the pharmaceutical compositions prepared in Examples 2 and 6 was measured by HPLC immediately after the compositions were prepared. HPLC was performed on a YMC-Pack ODS-AQ analytical column (100x4.6 mm I.D.), packed with 3µm diameter particles (YMC SEPARATION TECHNOLOGY). The mobile phase was a mixture of acetonitrile and 20 mM KH₂PO₄ at pH 2.0 (60:40) and its flow-rate was 1.0 mL/min. The UV detector was set at 285 nm and the column temperature was 30°C. The results are shown in Table 3.

**Table 3: Stability of Montelukast Sodium Compositions**

| Sample | % Sulfoxide of Formula (I) |
|---|---|
| Composition of Example 2 | 0.99 |
| Composition of Example 6 | 0.05 |

As can be seen from the data presented, the formulations manufactured by wet granulation (Examples 2 and 5) are less stable than those manufactured from a dry-mix of ingredients (Examples 3 and 6).

### Example 8: (Reference)

A pharmaceutical composition of montelukast sodium chewable tablets was prepared by wet granulation. A mixture was made of montelukast sodium (42.1 g), hydroxypropyl cellulose (40 g), sodium starch glycolate (96 g), mannitol granular (512 g), color iron oxide (4 g), aspartame (4 g), sodium lauryl sulfate (14.4g) and flavor (32 g). The mixture was then granulated using purified water as a granulating liquid to form a granulate. The granulate was dried, milled and blended with magnesium stearate (16 g) to form a blend. The blend was compressed into chewable tablets.

## Claims

1. A stable pharmaceutical composition in film coated tablet form comprising montelukast or a salt thereof and a pharmaceutically acceptable excipient selected from at least one of diluent, binder, disintegrant, wetting agent, lubricant, or glidant, provided that the pharmaceutically acceptable excipient is not microcrystalline cellulose, wherein the pharmaceutical composition is prepared by direct compression from a dry mix..

2. The pharmaceutical composition of claim 1, wherein the composition contains the corresponding montelukast sulfoxide wherein the corresponding montelukast sulfoxide in the composition does not increase by more than 1% by weight from the initial amount of montelukast after storage at about 40°C and about 75% relative humidity for 3 months.

3. The pharmaceutical composition of claim 2, wherein the salt is montelukast sodium and the corresponding sulfoxide is the sulfoxide of formula (I):

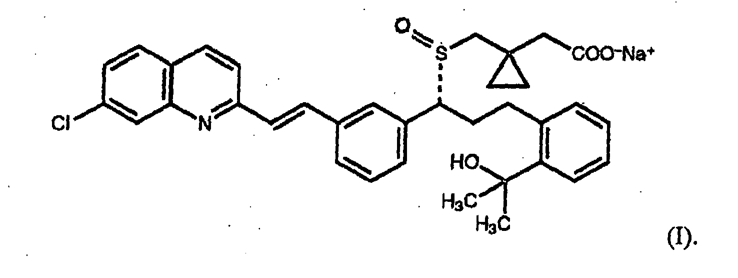

4. The pharmaceutical composition of claim 2 or claim 3, wherein the amount of the corresponding sulfoxide does not increase by more than 0.5% by weight of the initial amount of montelukast after storage at about 40°C and about 75% relative humidity for 3 months.

5. The pharmaceutical composition of claim 4, wherein the amount of the corresponding sulfoxide does not increase by more than 0.3% by weight of the initial amount of montelukast after storage at about 40°C and about 75% relative humidity for 3 months.

6. The pharmaceutical composition of claim 5, wherein the amount of the corresponding sulfoxide does not increase by more than 0.1% by weight of the initial amount of montelukast after storage at about 40°C and about 75% relative humidity for 3 months.

7. A pharmaceutical composition in film coated tablet form comprising montelukast or a salt thereof and a pharmaceutically acceptable excipient, provided that the pharmaceutically acceptable excipient is not microcrystalline cellulose, wherein the corresponding montelukast sulfoxide is present in an amount of not more than 0.2% by weight of montelukast or a salt thereof immediately after preparation, wherein the pharmaceutical composition is prepared by direct compression from a dry mix.

8. The pharmaceutical composition of claim 7, wherein the corresponding sulfoxide is present in an amount of not more than 0.1% by weight of montelukast immediately after preparation.

9. The pharmaceutical composition of claim 7 or claim 8, wherein the salt is montelukast sodium and the corresponding sulfoxide is the sulfoxide of formula (I):

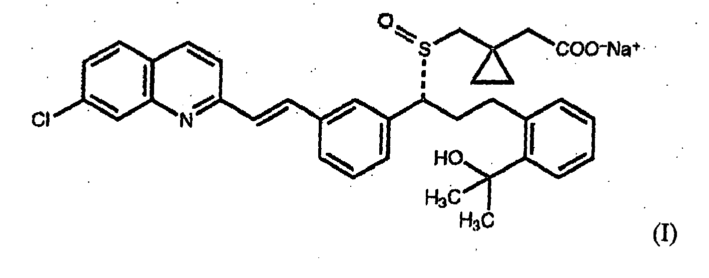

10. The pharmaceutical composition of any one of the preceding claims, wherein the composition comprises a wetting agent.

11. The pharmaceutical composition of claim 10, wherein the wetting agent is sodium lauryl sulfate.

12. The pharmaceutical composition of any of claims 1 to 9, wherein the tablet is a film coated tablet further comprising a coating agent, provided that the coating agent is not microcrystalline cellulose.

13. The pharmaceutical composition of claim 12, comprising montelukast sodium, lactose monohydrate, hydroxypropylcellulose, starch, sodium starch glycolate and magnesium stearate.

14. The pharmaceutical composition of claim 13, wherein the montelukast sodium is present in an amount of about 5% by weight, the lactose monohydrate is present in an amount of about 62% by weight, the hydroxypropylcellulose is present in an amount of about 2% by weight, the starch is present in an amount of about 18% by weight, the sodium starch glycolate is present in an amount of about 9% by weight, the magnesium stearate is present in an amount of about 1% by weight, and the coating agent is present in an amount of about 3% by weight.

15. The pharmaceutical composition of claim 12, comprising montelukast sodium, lactose monohydrate, hydroxypropylcellulose, starch, sodium lauryl sulfate, sodium starch glycolate and magnesium stearate.

16. A process for preparing a pharmaceutical composition in film coated tablet form as defined in any one of the preceding claims, comprising combining montelukast or a pharmaceutically acceptable salt thereof with a pharmaceutically acceptable excipient selected from at least one of diluent, disintegrant, wetting agent, lubricant, or glidant, provided that the pharmaceutically acceptable excipient is not microcrystalline cellulose.

17. The process of claim 16, wherein each of the pharmaceutically acceptable excipients is first tested in order to assess its suitability in a stable montelukast pharmaceutical composition.

18. The process of claim 16 for preparing a pharmaceutical composition as defined in claim 15 comprising blending montelukast sodium, lactose monohydrate, hydroxypropylcellulose, starch, sodium lauryl sulfate, sodium starch glycolate and magnesium stearate in a dry mixing method and compressing the blended components into tablet form.

## Patentansprüche

1. Stabile pharmazeutische Zusammensetzung in Form einer Filmtablette, die Montelukast oder ein Salz davon und ein pharmazeutisch verträgliches Bindemittel umfasst, ausgewählt unter wenigstens einem unter einem Verdünnungsmittel, Binder, Sprengmittel, Benetzungsmittel, Schmiermittel oder Fliesregulierungsmittel, unter der Voraussetzung, dass das pharmazeutisch verträgliche Bindemittel nicht mikrokristalline Zellulose ist, wobei die pharmazeutische Zusammensetzung durch direktes Komprimieren aus einem trockenen Gemisch hergestellt wird.

2. Pharmazeutische Zusammensetzung gemäß Anspruch 1, wobei die Zusammensetzung das entsprechende Montelukast-Sulfoxid enthält, wobei das entsprechende Montelukast-Sulfoxid in der Zusammensetzung nach Lagerung bei ungefähr 40°C und ungefähr 75% relativer Feuchte für 3 Monate nicht um mehr als 1 Gew.-% der anfänglichen Menge des Montelukast zunimmt.

3. Pharmazeutische Zusammensetzung gemäß Anspruch 2, wobei das Salz Montelukast-Natrium ist und das entsprechende Sulfoxid das Sulfoxid der Formel (I) ist:

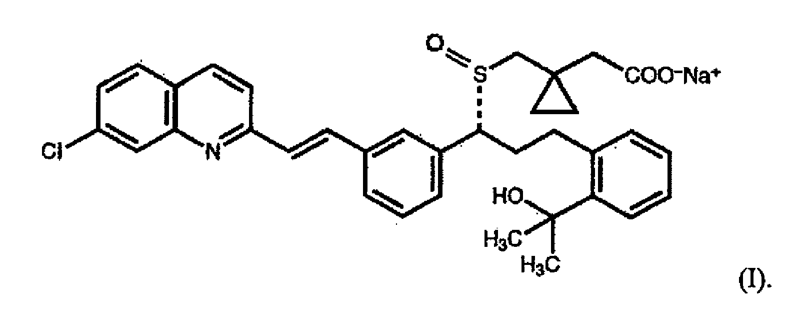

4. Pharmazeutische Zusammensetzung gemäß Anspruch 2 oder 3, wobei die Menge des entsprechenden Sulfoxids nach Lagerung bei ungeführ 40°C und ungefähr 75% relativer Feuchte für 3 Monate nicht um mehr als 0,5 Gew.-% der anfänglichen Menge des Montelukast zunimmt.

5. Pharmazeutische Zusammensetzung nach Anspruch 4, wobei die Menge des entsprechenden Sulfoxids nach Lagerung bei ungefähr 40°C und ungefähr 75% relativer Feuchte für 3 Monate nicht um mehr als 0,3 Gew.-% der anfänglichen Menge des Montelukast zunimmt.

6. Pharmazeutische Zusammensetzung nach Anspruch 5, wobei die Menge des entsprechenden Sulfoxids nach Lagerung bei ungefähr 40°C und ungefähr 75% relativer Feuchte für 3 Monate nicht um mehr als 0,1 Gew.-% der anfänglichen Menge des Montelukast zunimmt.

7. Pharmazeutische Zusammensetzung in Form einer Filmtablette, die Montelukast oder einer Salz davon und ein pharmazeutisch verträgliches Bindemittel umfasst, unter der Voraussetzung, dass das pharmazeutisch verträgliche Bindemittel nicht mikrokristalline Zellulose ist, wobei das entsprechende Montelukast-Sulfoxid in einer Menge vorhanden ist, die nicht mehr als 0,2 Gew.-% des Montelukast oder Salz davon unmittelbar nach der Herstellung beträgt, wobei die pharmazeutische Zusammensetzung durch direktes Komprimieren aus einem trockenen Gemisch hergestellt wird.

8. Pharmazeutische Zusammensetzung nach Anspruch 7, wobei das entsprechende Sulfoxid in einer Menge von nicht mehr als 0,1 Gew.-% des Montelukast unmittelbar nach der Herstellung vorhanden ist.

9. Pharmazeutische Zusammensetzung gemäß Anspruch 7 oder 8, wobei das Salz Montelukast-Natrium ist und das entsprechende Sulfoxid das Sulfoxid mit der Formel (I) ist:

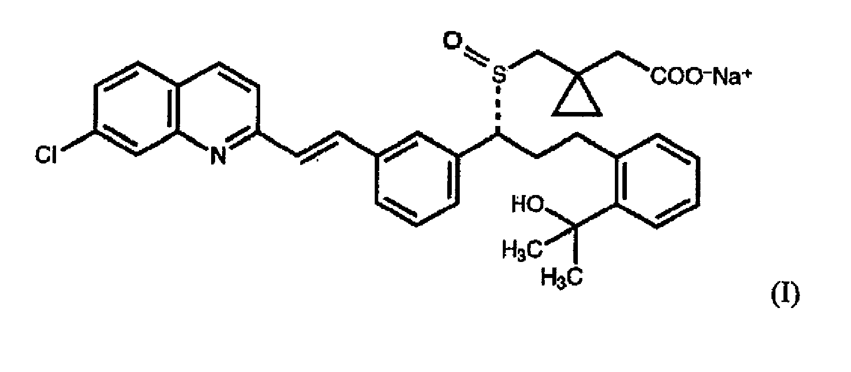

10. Pharmazeutische Zusammensetzung gemäß einem der vorangehenden Ansprüche, wobei die Zusammensetzung ein Benetzungsmittel umfasst.

11. Pharmazeutische Zusammensetzung gemäß Anspruch 10, wobei das Benetzungsmittel Natriumlaurylsulfat ist.

12. Pharmazeutische Zusammensetzung gemäß einem der Ansprüche 1 bis 9, wobei die Tablette eine Filmtablette ist, die weiter ein Beschichtungsmittel umfasst, unter der Voraussetzung, dass das Beschichtungsmittel nicht mikrokristalline Zellulose ist.

13. Pharmazeutische Zusammensetzung gemäß Anspruch 12, die Montelukast-Natrium, Laktosemonohydrat, Hydroxypropylzellulose, Stärke, Natriumstärkeglykolat und Magnesiumstearat umfasst.

14. Pharmazeutische Zusammensetzung gemäß Anspruch 13, wobei das Montelukast-Natrium in einer Menge von ungefähr 5 Gew.-% vorhanden ist, das Laktosemonohydrat in einer Menge von ungefähr 62 Gew.-% vorhanden ist, die Hydroxypropylzellulose in einer Menge von ungefähr 2 Gew.-% vorhanden ist, die Stärke in einer Menge von ungefähr 18 Gew.-% vorhanden ist, das Natriumstärkeglykolat in einer Menge von ungefähr 9 Gew.-% vorhanden ist, das Magnesiumstearat in einer Menge von ungefähr 1 Gew.-% vorhanden ist und das Beschichtungsmittel in einer Menge von ungefähr 3 Gew.-% vorhanden ist.

15. Pharmazeutische Zusammensetzung gemäß Anspruch 12, die Montelukast-Natrium, Laktosemonohydrat, Hydroxypropylzellulose, Stärke, Natriumlaurylsulfat, Natriumstärkeglykolat und Magnesiumstearat umfasst.

16. Verfahren zur Herstellung einer pharmazeutischen Zusammensetzung in Form einer Filmtablette, wie sie in einem der vorangehenden Ansprüche definiert ist, bei dem Montelukast oder ein pharmazeutisch verträgliches Salz davon mit einem pharmazeutisch verträglichen Bindemittel zusammengegeben wird, das unter wenigstens einem unter einem Verdünnungsmittel, Sprengmittel, Benetzungsmittel, Schmiermittel, Fließregulierungsmittel ausgewählt ist, unter der Voraussetzung, dass das pharmazeutisch verträgliche Bindemittel nicht mikrokristalline Zellulose ist.

17. Verfahren nach Anspruch 16, wobei jedes der pharmazeutisch verträglichen Bindemittel zunächst untersucht wird, um dessen Geeignetheit in einer stabilen pharmazeutischen Zusammensetzung mit Montelukast zu bewerten.

18. Verfahren nach Anspruch 16 zur Herstellung einer pharmazeutischen Zusammensetzung, wie sie in Anspruch 15 definiert ist, bei dem Montelukast-Natrium, Laktosemonohydrat, Hydroxypropylzellulose, Stärke, Natriumlaurylsulfat, Natriumstärkeglykolat und Magnesiumstearat in einem Trockenmischverfahren vermengt werden und die vermengten Komponenten in Tablettenform gepresst werden.

## Revendications

1. Composition pharmaceutique stable sous forme de comprimé pelliculé comprenant du montelukast ou un sel de celui-ci et un excipient pharmaceutiquement acceptable choisi parmi au moins un diluant, un liant, un délitant, un agent mouillant, un lubrifiant ou d'un agent de glissement, à condition que l'excipient pharmaceutiquement acceptable ne soit pas de la cellulose microcristalline, dans laquelle la composition pharmaceutique est préparée par compression directe à partir d'un mélange sec.

2. Composition pharmaceutique selon la revendication 1, dans laquelle la composition contient le sulfoxyde de montelukast correspondant dans laquelle le sulfoxyde de montelukast correspondant dans la composition n'augmente pas de plus de 1 % en poids par rapport à la quantité initiale de montelukast après stockage à environ 40 °C et environ 75 % d'humidité relative pendant 3 mois.

3. Composition pharmaceutique selon la revendication 2, dans laquelle le sel est le montelukast sodique et le sulfoxyde correspondant est le sulfoxyde de formule (I) :

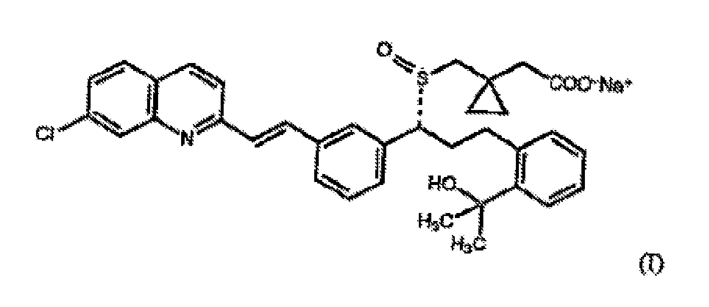

4. Composition pharmaceutique selon la revendication 2 ou la revendication 3, dans laquelle la quantité de sulfoxyde correspondant n'augmente pas de plus de 0,5 % en poids de la quantité initiale de montelukast après stockage à environ 40 °C et environ 75 % d'humidité relative pendant 3 mois.

5. Composition pharmaceutique selon la revendication 4, dans laquelle la quantité de sulfoxyde correspondant n'augmente pas de plus de 0,3 % en poids de la quantité initiale de montelukast après stockage à environ 40 °C et environ 75 % d'humidité relative pendant 3 mois.

6. Composition pharmaceutique selon la revendication 5, dans laquelle la quantité du sulfoxyde correspondant n'augmente pas de plus de 0,1 % en poids de la quantité initiale de montelukast après stockage à environ 40 °C et environ 75 % d'humidité relative pendant 3 mois.

7. Composition pharmaceutique stable sous forme de comprimé pelliculé comprenant du montelukast ou un sel de celui-ci et un excipient pharmaceutiquement acceptable, à condition que l'excipient pharmaceutiquement acceptable ne soit pas de la cellulose microcristalline, dans laquelle le sulfoxyde de montelukast correspondant est présent en une quantité non supérieure à 0,2 % en poids du montelukast ou d'un sel de celui-ci immédiatement après préparation, dans laquelle la composition pharmaceutique est préparée par compression directe à partir d'un mélange sec.

8. Composition pharmaceutique selon la revendication 7, dans laquelle le sulfoxyde correspondant est présent dans une quantité non supérieure à 0,1 % en poids du montelukast immédiatement après préparation.

9. Composition pharmaceutique selon la revendication 7 ou revendication 8, dans laquelle le sel est le montelukast sodique et le sulfoxyde correspondant est le sulfoxyde de formule (I) :

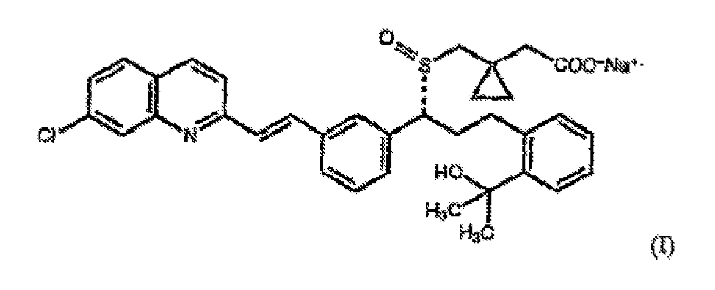

10. Composition pharmaceutique selon l'une quelconque des revendications précédentes, dans laquelle la composition comprend un agent mouillant.

11. Composition pharmaceutique selon la revendication 10, dans laquelle l'agent mouillant est le lauryl sulfate de sodium.

12. Composition pharmaceutique selon l'une quelconque des revendications 1 à 9, dans laquelle le comprimé est un comprimé pelliculé comprenant en outre un agent d'enrobage, à condition que l'agent d'enrobage ne soit pas de la cellulose microcristalline.

13. Composition pharmaceutique selon la revendication 12, comprenant du montelukast sodique, du monohydrate de lactose, de l'hydroxypropylcellulose, de l'amidon, de l'amidon glycolate de sodium et du stéarate de magnésium.

14. Composition pharmaceutique selon la revendication 13, dans laquelle le montelukast sodique est présent dans une quantité d'environ 5 % en poids, le monohydrate de lactose est présent dans une quantité d'environ 62 % en poids, l'hydroxypropylcellulose est présente dans une quantité d'environ 2 % en poids, l'amidon est présent dans une quantité d'environ 18 % en poids, l'amidon glycolate de sodium est présent dans une quantité d'environ 9 % en poids, le stéarate de magnésium est présent dans une quantité d'environ 1 % en poids et l'agent d'enrobage est présent dans une quantité d'environ 3 % en poids.

15. Composition pharmaceutique selon la revendication 12, comprenant du montelukast sodique, du monohydrate de lactose, de l'hydroxypropylcellulose, de l'amidon, de lauryl sulfate de sodium, de l'amidon glycolate de sodium et du stéarate de magnésium.

16. Procédé de préparation d'une composition pharmaceutique sous forme de comprimé pelliculé selon l'une quelconque des revendications précédentes, comprenant la combinaison de montelukast ou d'un sel pharmaceutiquement acceptable de celui-ci et d'un excipient pharmaceutiquement acceptable choisi parmi au moins l'un d'un diluant, d'un délitant, d'un agent mouillant, d'un lubrifiant ou d'un agent de glissement, à condition que l'excipient pharmaceutiquement acceptable ne soit pas la cellulose microcristalline.

17. Procédé selon la revendication 16, dans lequel chacun des excipients pharmaceutiquement acceptables est d'abord évalué afin d'estimer son caractère approprié dans une composition pharmaceutique de montelukast stable.

18. Procédé selon la revendication 16, pour la préparation d'une composition pharmaceutique selon la revendication 15, comprenant le fait de mélanger du montelukast sodique, du monohydrate de lactose, de l'hydroxypropylcellulose, d'amidon, du lauryl sulfate de sodium, de l'amidon glycolate de sodium et du stéarate de magnésium dans un procédé de mélange à sec et la compression des composants mélangés sous forme de comprimé.
